# EUROPÄISCHE PATENTSCHRIFT

(11) **EP 3 632 887 B1**
(45) Veröffentlichungstag und Bekanntmachung des Hinweises auf die Patenterteilung: **14.04.2021**
(21) Anmeldenummer: 19200448.9
(22) Anmeldetag: 30.09.2019
(51) Int. Cl.: C07C 45/50, B01J 21/08, B01J 23/46, B01J 31/02, B01J 31/16, B01J 31/18, B01J 35/04, B01J 37/02, B01J 19/24

(54) **VERFAHREN ZUM ANFAHREN EINES HYDROFORMYLIERUNGSREAKTORS**
PROCESS FOR STARTING A HYDROFORMYLATION REACTOR
PROCÉDÉ DE DÉMARRAGE D'UN RÉACTEUR D'HYDROFORMYLATION

(30) Priorität: 05.10.2018 EP 18198794
(43) Veröffentlichungstag der Anmeldung: 08.04.2020
(73) Patentinhaber: Evonik Operations GmbH, 45128 Essen (DE)
(72) Erfinder: Haßelberg, Jennifer, 44139 Dortmund (DE); Franke, Robert, 45772 Marl (DE); Stenger, Frank, 63755 Alzenau (DE); Kreis, Peter, 44227 Dortmund (DE); Hecht, Corinna, 45721 Haltern am See (DE); Kristen, Marc Oliver, 45721 Haltern am See (DE); Ott, Florian, 45657 Recklinghausen (DE)
(74) Vertreter: Evonik Patent Association

(56) Entgegenhaltungen:
- WO-A1-2015/028284
- GB-A- 1 335 531

## Beschreibung

Die vorliegende Erfindung betrifft ein Verfahren zum schrittweisen Anfahren eines Hydroformylierungsreaktors, also zur schrittweisen Überführung eines Reaktors mit einem neu eingebrachten oder regenerierten Hydroformylierungskatalysator in den Betrieb.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Um Problemen bei der homogen katalysierten Hydroformylierung vorzubeugen, sind Hydroformylierungsverfahren entwickelt worden, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

Im Hinblick auf die Immobilisierung bzw. Heterogenisierung offenbart die bereits erwähnte WO 2015/028284 A1 sogenannte SILP-Systeme (SILP = Supported lonic Liquid Phase), bei der das Katalysatorsystem mit Rhodium, Iridium oder Cobalt als Zentralatom insbesondere auf einem porösen Siliciumdioxid-Träger unter Verwendung einer ionischen Flüssigkeit immobilisiert wird. Nach Herstellung eines frischen Hydroformylierungskatalysators oder nach der Regeneration eines gebrauchten Hydroformylierungskatalysators weist der Katalysator aufgrund der Vielzahl an katalytisch aktiven Zentren typischerweise eine besonders hohe Aktivität für die zu katalysierende Reaktion auf. Erst mit der Zeit werden die katalytisch aktiven Zentren beispielsweise durch Nebenproduktbildung deaktiviert und die Katalysatoraktivität pendelt sich ein.

Das Problem dabei ist, dass durch die hohe Startaktivität des Hydroformylierungskatalysators ein Umsatzmaximum für die Hydroformylierung auftritt, was auch eine maximale Bildung von Nebenprodukten zur Folge hat. Übersteigt der Anteil der zumeist hochsiedenen Nebenprodukte einen gewissen Wert kann es zur Ausbildung einer Flüssigphase kommen, die den Hydroformylierungskatalysator desaktivieren bzw. schädigen oder durch die der Katalysator ausgewaschen werden kann.

Die Aufgabe der vorliegenden Erfindung bestand deshalb darin, ein Verfahren zum Anfahren eines Hydroformylierungsreaktors bereitzustellen, welches die vorgenannten Probleme nicht aufweist und insbesondere zu einer Erhöhung des Umsatzes sowie der Lebenszeit des Katalysators führt.

Gelöst wird diese Aufgabe gemäß Anspruch 1 dadurch, dass der Anteil an Olefinen in dem Einsatzgemisch und/oder der Anteil an Synthesegas in dem Synthesegasgemisch in mehreren Schritten erhöht wird, der Umsatz einen bestimmten Maximalwert aber nicht überschreitet. Die Aktivierung des Katalysators durch das erfindungsgemäße Anfahren wird so mit einer zeitlich verlängerten Umsatzsteigerung realisiert.

Ein Gegenstand der vorliegenden Erfindung ist somit ein Verfahren zum Anfahren eines Hydroformylierungsreaktors, der ein auf einem Support heterogenisiertes Hydroformylierungskatalysatorsystem enthält, wobei das Hydroformylierungskatalysatorsystem ein Metall aus der 8. oder 9. Gruppe des Periodensystems der Elemente, mindestens einen organischen Phosphor-enthaltenden Liganden, einen Stabilisator und optional eine ionische Flüssigkeit umfasst, wobei der Support ein Monolith, das heißt ein Block aus dem porösen keramischen Material ist, und wobei das Verfahren dadurch gekennzeichnet ist, dass
ein gasförmiges Einsatzgemisch, welches zu hydroformylierende C2- bis C8-Olefine umfasst, zusammen mit einem Synthesegasgemisch in den Reaktor eingeleitet wird, und
die Zusammensetzung des Einsatzgemisches und/oder die Zusammensetzung des Synthesegasgemisches bei konstantem Volumenstrom in mehreren Schritten dadurch variiert wird, dass der Anteil an zu hydroformylierenden C2- bis C8-Olefinen im Einsatzgemisch und/oder der Anteil von Synthesegas im Synthesegasgemisch schrittweise erhöht wird,
mit der Maßgabe, dass ein maximaler Umsatz der eingesetzten C2- bis C8-Olefine von 40 bis 90% während des gesamten Anfahrens nicht überschritten wird.

Mit dem erfindungsgemäßen Verfahren kann eine schonende Aktivierung und eine Abfederung der maximalen Startaktivität des Hydroformylierungskatalysators erreicht werden. Das führt zu einer verlängerten Lebenszeit des Katalysators, sowie zur partiellen oder vollständigen Verhinderung der Flüssigphasenbildung, die eine Desaktivierung oder das Auswaschen des Katalysatorsystems zur Folge haben kann.

Als Einsatzgemisch für das erfindungsgemäße Verfahren können alle Gemische eingesetzt werden, die C2- bis C8-Olefine, vorzugsweise C2- bis C5-Olefine, insbesondere Ethen, Propen, 1-Buten, 2-Buten, Isobuten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Dazu gehören insbesondere technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Das Synthesegasgemisch gemäß der vorliegenden Erfindung besteht aus Synthesegas für die Hydroformylierung, enthält also Wasserstoff und Kohlenmonoxid, vorzugsweise in einem molaren Verhältnis von 1 : 1, und optional geringe Mengen an Verunreinigungen. Das Synthesegas kann durch dem Fachmann bekannte Verfahren erzeugt und bereitgestellt werden.

Die Erfindung sieht vor, dass entweder der Anteil des Synthesegases im Synthesegasgemisch oder der Anteil der Olefine im Einsatzgemisch durch schrittweise Erhöhung variiert werden. Die Variation der Anteile von Synthesegas und Olefinen können auch zusätzlich zueinander erfolgen, wobei die Anzahl der Schritte bei der Erhöhung der Anteile auch unterschiedlich voneinander sein kann. Zu Beginn sollte der Anteil des Synthesegases und/oder der Anteil der Oleine geringer sein, als im üblichen Einsatz bei der Hydroformylierung und dann sukzessive gesteigert werden bis nach vollständigem Anfahren und Übergang zu Hydroformylierung die finale(n) Zusammensetzung(en) erreicht ist.

Die Erhöhung des Anteils der Olefine am Einsatzgemisch und/oder der Anteil von Synthesegas am Synthesegasgemisch erfolgt in mehreren Schritten, also in mindestens zwei Schritten. In einer bevorzugten Ausführungsform erfolgt die Erhöhung Anteils der Olefine am Einsatzgemisch und/oder der Anteil von Synthesegas am Synthesegasgemisch in mindestens drei Schritten, besonders bevorzugt in mindestens 4 Schritten.

Um eine Variation des Anteils der Olefine im Einsatzgemisch und/oder des Anteils an Synthesegas im Synthesegasgemisch erreichen zu können, kann anfänglich ein Inertgas zum Einsatzgemisch und/oder zum Synthesegasgemisch hinzugefügt werden, um das Einsatzgemisch und/oder das Synthesegasgemisch zu verdünnen.

Im ersten Schritt wird vorzugsweise so viel Inertgas zum Einsatzgemisch und/oder zum Synthesegasgemisch hinzugefügt wird, dass der Anteil des Inertgases im Einsatzgemisch und/oder im Synthesegasgemisch im Bereich von 70% bis 90% liegt. Der Anteil des Inertgases im Einsatzgemisch und/oder im Synthesegasgemisch wird dann in dem folgenden Schritt oder in den folgenden Schritten sukzessive reduziert. In dem letzten Schritt des Anfahrens beträgt der Anteil des Inertgases im Einsatzgemisch und/oder im Synthesegasgemisch dann nur noch 10% bis 30%. Als Inertgase können Edelgase, insbesondere Argon oder Helium, C2- bis C8-Alkane, vorzugsweise C2- bis C5-Alkane oder Stickstoff eingesetzt werden.

Nach dem letzten Schritt wird die Zugabe des Inertgases vollständig gestoppt. Einsatzgemisch und/oder Synthesegasgemisch werden dann nicht mehr verdünnt, sondern werden in ihrer normalen, unverdünnten Zusammensetzung eingesetzt. Dadurch ist die Anfahrprozedur beendet und der Reaktor ist wieder im Normalbetrieb.

Der Support besteht in einer bevorzugten Ausführungsform der vorliegenden Erfindung aus einem keramischen Material. Das keramische Material ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon.

Die Silikatkeramik ist vorzugsweise ausgewählt aus Alumosilikat, Magnesiumsilikat und Mischungen davon, wie z. B. Betonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Siliciumnitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung besteht vorzugsweise aus einer carbidischen Keramik.

Auf das keramische Material kann optional zusätzlich ein sogenannter Washcoat aus dem gleichen oder einem unterschiedlichen Keramikmaterial, das für den Support verwendet wird, insbesondere ein aus den vorgenannten Keramikmaterialen ausgewähltes Keramikmaterial, vorzugsweise Siliciumoxid, aufgetragen werden. Der Washcoat selber kann porös oder unporös sein. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports.

Der Support ist ein Monolith, das heißt der Support besteht aus einem Block, ist also ein dreidimensionales Objekt, als auch in Form eines Pulvers, in Form eines Granulats, in Form von Pellets oder in Form von sphärischen Partikeln vorliegen. In einer bevorzugten Ausführungsform ist der Support ein Monolith.

Der Monolith kann sowohl einstückig ausgebildet sein als auch aus mehreren, also mindestens zwei Einzelteilen bestehen, die zu einem Block zusammengefügt werden können und/oder miteinander fest oder lösbar verbunden sind. Der Support-Monolith ist vorzugsweise ein sich dreidimensional erstreckendes Bauteil, welches in seinem Querschnitt grundsätzliche beliebige geometrische Formen, beispielsweise rund, eckig, quadratisch o. ä., aufweisen kann. Das sich dreidimensional erstreckende Bauteil, welches als Support-Monolith eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des Reaktors strömen) auf.

Der so geformte Support-Monolith weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des Reaktors gegenüberliegt, einen Abschluss aufweisen bzw. der Kanal zu diesem Ende hin geschlossen ist. Der Support-Monolith kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Support-Monoliths insbesondere so zu wählen, dass die mechanische Stabilität nicht beeinträchtigt wird.

Darüber hinaus ist der Support aus dem keramischen Material porös, weist also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Support-Monolith zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Das Katalysatorsystem liegt erfindungsgemäß heterogenisiert auf einem Support- Monolith vor. Im Sinne der vorliegenden Erfindung ist der Ausdruck "heterogenisiert auf einem Support-Monolith" so zu verstehen, dass das Katalysatorsystem durch Ausbildung eines dünnen, festen oder flüssigen Films mithilfe des Stabilisators und/oder der ionischen Flüssigkeit auf der inneren und/oder äußeren Oberfläche eines festen Trägermaterials immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren und/oder Kanäle. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Support-Monoliths.

Das Hydroformylierungskatalysatorsystem umfasst ein Übergangsmetall aus der 8. oder 9. Gruppe des Periodensystems der Elemente, mindestens einen organischen Phosphor-enthaltenden Liganden, einen Stabilisator und optional eine ionische Flüssigkeit umfasst.

Das Übergangsmetall aus der 8. oder 9. Gruppe des Periodensystems der Elemente für das erfindungsgemäße Hydroformylierungskatalysatorsystem, ist vorzugweise Eisen, Ruthenium, Iridium, Ruthenium, Cobalt oder Rhodium, besonders bevorzugt Cobalt und Rhodium.

Der organische Phosphor-enthaltende Ligand für das erfindungsgemäße Hydroformylierungskatalysatorsystem weist vorzugsweise die allgemeine Formel (VI)

R'-A- R"-A- R''' (VI)

wobei R', R" und R''' jeweils organische Reste sind, mit der Maßgabe das R' und R''' nicht identisch sind, und beide A jeweils eine brückende -O-P(-O)₂-Gruppe sind, wobei zwei der drei Sauerstoffatome -O-jeweils an Rest R' und den Rest R''' gebunden sind. Die organischen Reste R', R" und R''' enthalten vorzugsweise keine endständige Trialkoxysilangruppe.

In einer bevorzugten Ausführungsform sind R', R" und R''' in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R''' nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Der Stabilisator für das erfindungsgemäße Hydroformylierungskatalysatorsystem ist vorzugsweise eine organische Aminverbindung, besonders bevorzugt eine organische Aminverbindung, die mindestens eine 2,2,6,6-Tetramethylpiperidineinheit nach Formel (I) enthält:

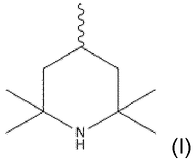

In einer besonders bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (I.5), (I.6), (I.7) und (I.8). 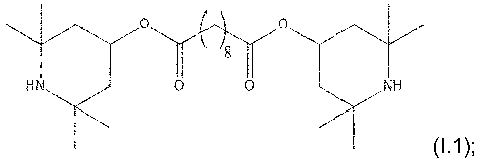 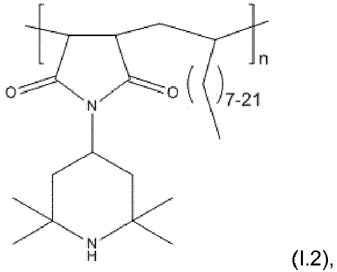 wobei n einer ganzen Zahl von 1 bis 20 entspricht; 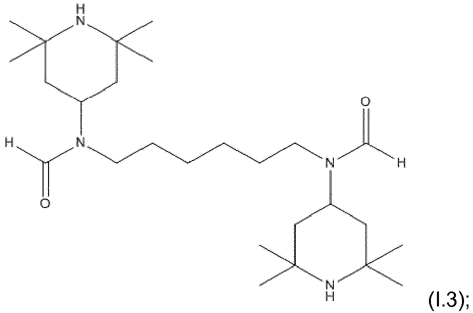 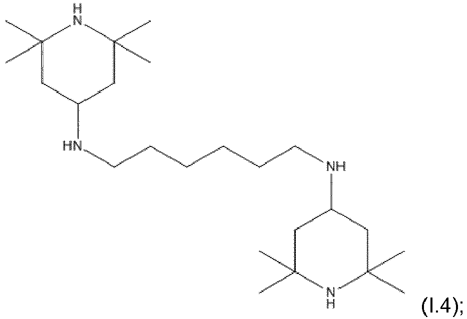 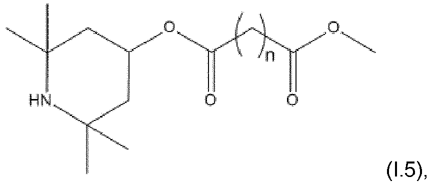 wobei n einer ganzen Zahl von 1 bis 12 entspricht; 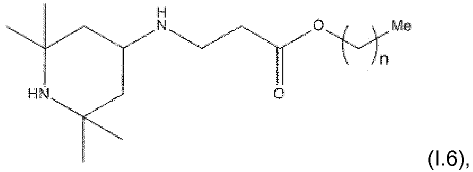 wobei n einer ganzen Zahl von 1 bis 17 entspricht; 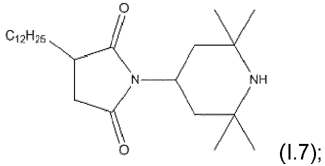 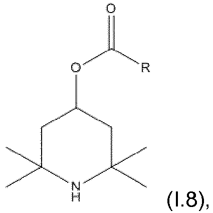 wobei R einer C6- bis C20-Alkylgruppe entspricht.

Die optional vorliegende ionische Flüssigkeit für das erfindungsgemäße Hydroformylierungskatalysatorsystem ist eine nahezu wasserfrei (Wassergehalt < 1,5 Gew.-% bezogen auf die gesamte ionische Flüssigkeit) Flüssigkeit, die bei Normaldruck (1,01325 bar) und vorzugsweise bei 25 °C flüssig ist. Die ionische Flüssigkeit besteht vorzugsweise zu mehr als 98 Gew.-% aus Ionen.

In einer bevorzugten Ausführungsform wird das Anion der ionischen Flüssigkeit ausgewählt aus der Gruppe, bestehend aus Tetrafluoroborat [BF4]⁻; Hexafluorophosphat [PF6]⁻; Dicyanamid [N(CN)₂]⁻; Bis(trifluoromethylsulfonyl)imid [NTf₂]⁻; Tricyanomethid [C(CN)₃]⁻; Tetracyanoborat [B(CN)₄]⁻; Halogeniden, insbesondere Cl⁻, Br⁻, F⁻, I⁻; Hexafluoroantimonat [SbF₆]⁻; Hexafluoroarsenat [AsF₆]⁻; Sulfat [SO₄]²⁻; Tosylat [C₇H₇SO₃]⁻; Triflat CF₃SO₃⁻; Nonaflat [C₄F₉SO₃]⁻; Tris-(Pentafluoroethyl)-trifluorophosphat [PF₃(C₂F₅)₃]⁻; Thiocyanat [SCN]⁻; Carbonat [CO₃]²⁻; [RA-COO]⁻; [RA-SO₃]⁻; [RA-SO₄]⁻; [RAPO₄RB]- und [(RA-SO₂)₂N]⁻, wobei RA und RB gleich oder ungleich voneinander sein können und jeweils eine lineare oder verzweigte aliphatischer oder alicyclischer Alkylgruppe mit 1 bis 12 Kohlenstofatomen, eine Perfluoralkylgruppe oder eine C5-C18-substituierte Arylgruppe ist, die durch ein oder mehrere Halogenatome substituiert ein kann.

Das Kation der ionischen Flüssigkeit wird vorzugsweise ausgewählt aus der Gruppe, bestehend aus quaternäre Ammonium-Kationen der allgemeinen Formel [NR¹R²R³R⁴]⁺ wobei R¹, R², R³, R⁴ jeweils unabhängig voneinander eine C1-C8-Alkylgruppe darstellen; Phosphonium-Kationen der allgemeinen Formel [PR¹R²R³R⁴]⁺ wobei R¹, R², R³, R⁴ jeweils unabhängig voneinander eine C1-C8-Alkylgruppe darstellen; Imidazolium-Kationen der allgemeinen Formel (II) 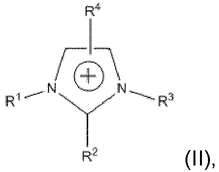 wobei R¹, R², R³ und R⁴ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyridinium-Kationen der allgemeinen Formel (III) 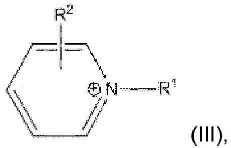 wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyrazolium-Kationen der allgemeinen Formel (IV) 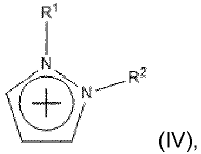 wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Triazolium-Kationen der allgemeinen Formel (V) 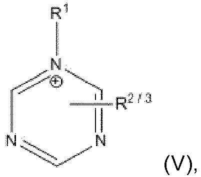 wobei R¹ und R² und/oder R³ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen.

In einer bevorzugten Ausführungsform ist das Kation der ionischen Flüssigkeit ein Imidazolium-Kation nach der vorgenannten allgemeinen Formel (II) mit entsprechender Definition der Reste R¹ bis R⁴. In einer besonders bevorzugten Ausführungsform ist die ionische Flüssigkeit ausgewählt aus der Gruppe, bestehend aus 1-Ethyl-3-methylimidazolium bis(trifluoromethylsulfonyl)imid, 1-Butyl-3-methylimidazolium hexafluorophosphat, 1-Butyl-3-methylimidazolium tetrafluoroborat, 1-Butyl-3-methylimidazolium bis(trifluoromethylsulfonyl)imid, 1-Ethyl-3-methylimidazolium ethylsulfat, Trioctyl-Methylammonium bis(trifluoromethylsulfonyl)imid und 1-Butyl-3-methylimidazolium octylsulfat.

Die optional vorhandene ionische Flüssigkeit kann als Trägerlösung für den Übergangsmetallkatalysator mit Liganden und den Stabilisator dienen. Dabei ist es wichtig, dass die ionische Flüssigkeit die Reaktanden (Einsatzolefine und Synthesegas) in ausreichendem Maße aufnehmen, d. h. lösen, kann und einen vergleichsweise niedrigen Dampfdruck aufweist, damit das Katalysatorsystem auch bei hohen Temperaturen als Flüssigkeitsreservoir vorliegt. Es konnte jedoch überraschenderweise herausgefunden werden, dass auch der Stabilisator einen stabilen Flüssigkeitsfilm in den Poren des Support-Monoliths ausbilden kann und damit in der Lage ist, die ionische Flüssigkeit teilweise oder vollständig zu ersetzen.

Für alle filmbildenden Komponenten, d. h. in diesem Fall die ionische Flüssigkeit und/oder der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Nach dem erfindungsgemäßen Anfahren des Hydroformylierungsreaktors befindet sich der Reaktor im Betrieb und wird zur Hydroformylierung von Olefinen verwendet. Die Hydroformylierung wird vorzugsweise bei den folgenden Bedingungen durchgeführt: Die Temperatur bei der Hydroformylierung sollte im Bereich von 65 bis 200 °C, vorzugsweise 75 bis 175 °C und besonders bevorzugt 85 bis 150 °C liegen. Der Druck sollte 35 bar, vorzugsweise 30 bar, besonders bevorzugt 25 bar während der Hydroformylierung nicht überschreiten. Das molare Verhältnis zwischen Synthesegas und dem zweiten Einsatzgemisch sollte zwischen 6:1 und 1:1 liegen.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

### Beispiel:

### Versuch 1: Herstellung und Untersuchung eines erfindungsgemäßen Katalysatorsystems

Als Support wurde ein Monolith aus Siliciumcarbid mit einer Länge von ca. 20 cm und einem Durchmesser von ca. 25 mm verwendet. Der Support war porös und wurde mit einem Washcoat (SiO₂) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Biphephos (Ligand), Bis(2,2,6,6-tetramethyl-4-piperidyl)sebacat (Stabilisator) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 19,14 |
| cis-2-Buten | 19,10 |
| trans-2-Buten | 28,40 |
| n-Butan | 30,80 |
| iso-Butan | 0,02 |
| 2-Methyl-butan | 2,50 |

Das Einsatzgemisch wurde zusammen mit Synthesegas (molares Verhältnis Synthesegas :
Einsatzgemisch = 3,5 : 1) zur Hydroformylierung mit einem Gasvolumenstrom von 390 ml/min in den Reaktor geleitet. Das Anfahren bestand aus insgesamt drei Stufen, wobei Einsatzgemisch und Synthesegas mit Stickstoff so verdünnt wurden, dass in der ersten Stufe 75% des Gasvolumenstroms (also 290 ml/min) aus Stickstoff bestanden, in der zweite Stufe ca. 50% des Gasvolumenstroms (ca. 187 ml/min) aus Stickstoff bestanden, in der dritten Stufe ca. 25% des Gasvolumenstroms aus Stickstoff bestanden. Nach der dritten Stufe wurde kein Stickstoff mehr zugegeben und der Reaktor wurde dadurch vollständig mit Einsatzgemisch und Synthesegas belastet.

Die Hydroformylierung wurde in allen Stufen und im Betrieb bei einer Temperatur von 120 °C und einem Druck von 10 bar durchgeführt.

Im Vergleich zu einem Versuch, bei dem der Reaktor direkt mit einem Gasvolumenstrom von 390 ml/min und ohne Verdünnung mit Stickstoff belastet worden ist, konnte herausgefunden werden, dass die Lebensdauer des Katalysators in etwa verdoppelt werden konnte, wenn die erfindungsgemäße Anfahrprozedur verwendet wird.

Versuch 2: Herstellung und Untersuchung eines erfindungsgemäßen Katalysatorsystems Als Ausgangsmaterial für den Support wurde ein Monolith aus Siliciumcarbid mit einer Länge von ca. 20 cm und einem Durchmesser von ca. 25 mm verwendet. Dieser Monolith wurde mit einem Backenbrecher mit einem Walzenspalt von 2 mm zerkleinert. Der zerkleinerte Support wurde dann auf ein Zielkorn von 2 bis 3,15 mm abgesiebt mit einem Washcoat (SiO₂) vorbehandelt. Das so hergestellte Granulat wurde anschließend in eine 20 cm lange runde Reaktorhülse mit einem Durchmesser von einem Zoll (ca. 2,54 cm) eingebracht, wobei ober- und unterhalb des Granulats Glasperlen ähnlicher Größe eingefüllt wurden. Das Granulat wurde dann mit einer Katalysatorlösung, enthaltend Rh(acac)(CO)₂, Bisphephos (Ligand), Bis(2,2,6,6-tetramethyl-4-piperidyl)sebacat (Stabilisator) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen, wurde das auf dem Supportgranulat heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 19,14 |
| cis-2-Buten | 19,10 |
| trans-2-Buten | 28,40 |
| n-Butan | 30,80 |
| iso-Butan | 0,02 |
| 2-Methyl-butan | 2,50 |

Das Einsatzgemisch wurde zusammen mit Synthesegas (molares Verhältnis Synthesegas :
Einsatzgemisch = 3,5 : 1) zur Hydroformylierung mit einem Gasvolumenstrom von 390 ml/min in den Reaktor geleitet. Das Anfahren bestand aus insgesamt drei Stufen, wobei Einsatzgemisch und Synthesegas mit Stickstoff so verdünnt wurden, dass in der ersten Stufe 75% des Gasvolumenstroms (also 290 ml/min) aus Stickstoff bestanden, in der zweite Stufe ca. 50% des Gasvolumenstroms (ca. 187 ml/min) aus Stickstoff bestanden, in der dritten Stufe ca. 25% des Gasvolumenstroms aus Stickstoff bestanden. Nach der dritten Stufe wurde kein Stickstoff mehr zugegeben und der Reaktor wurde dadurch vollständig mit Einsatzgemisch und Synthesegas belastet.

Auch hier zeigte sich im Vergleich zu einem Versuch, bei dem der Reaktor direkt mit einem Gasvolumenstrom von 390 ml/min und ohne Verdünnung mit Stickstoff belastet worden ist, dass die Lebensdauer des Katalysators durch die erfindungsgemäße Anfahrprozedur vergrößert werden kann.

## Patentansprüche

1. Verfahren zum Anfahren eines Hydroformylierungsreaktors, der ein auf einem Support-Monolith heterogenisiertes Hydroformylierungskatalysatorsystem enthält, wobei das Hydroformylierungskatalysatorsystem ein Metall aus der 8. oder 9. Gruppe des Periodensystems der Elemente, mindestens einen organischen Phosphor-enthaltenden Liganden, einen Stabilisator und optional eine ionische Flüssigkeit umfasst, wobei das Verfahren **dadurch gekennzeichnet ist, dass**
ein gasförmiges Einsatzgemisch, welches zu hydroformylierende C2- bis C8-Olefine umfasst, zusammen mit einem Synthesegasgemisch in den Reaktor eingeleitet wird, und die Zusammensetzung des Einsatzgemisches und/oder die Zusammensetzung des Synthesegasgemisches bei konstantem Volumenstrom in mehreren Schritten dadurch variiert wird, dass der Anteil an zu hydroformylierenden C2- bis C8-Olefinen im Einsatzgemisch und/oder der Anteil von Synthesegas im Synthesegasgemisch schrittweise erhöht wird,
mit der Maßgabe, dass ein maximaler Umsatz der eingesetzten C2- bis C8-Olefine von 40 bis 90% während des gesamten Anfahrens nicht überschritten wird.

2. Verfahren nach Anspruch 1, wobei dem Einsatzgemisch und/oder dem Synthesegasgemisch ein Inertgas zur Verringerung des Anteils an C2- bis C8-Olefinen und/oder zur Verringerung des Anteils an Synthesegas hinzugefügt und die Zugabe des Inertgases entsprechend der schrittweisen Erhöhung des Anteils an C2- bis C8-Olefinen und/oder des Anteils an Synthesegas schrittweise reduziert wird.

3. Verfahren nach Anspruch 1 oder 2, wobei so viel Inertgas zum Einsatzgemisch und/oder zum Synthesegasgemisch hinzugefügt wird, dass der Anteil des Inertgases im Einsatzgemisch und/oder im Synthesegasgemisch im Bereich von 70% bis 90% liegt.

4. Verfahren nach Anspruch 3, wobei das Inertgas ein Edelgas, insbesondere Argon oder Helium, ein C2- bis C8-Alkan oder Stickstoff ist.

5. Verfahren nach einem der Ansprüche 1 bis 4, wobei der Support-Monolith aus einem porösen keramischen Material besteht, welches aus der Gruppe, bestehend aus einer Silikatkeramik, einer oxidischen Keramik, einer nitridischen Keramik, einer carbidischen Keramik, einer silicidischen Keramik und Mischungen davon, ausgewählt wird.

6. Verfahren nach Anspruch 5, wobei das poröse keramische Material, aus dem der Support-Monolith besteht, eine carbidische Keramik ist.

7. Verfahren nach Anspruch 1, wobei der Support-Monolith einen oder mehrere, vorzugsweise durchgängige Kanäle in Hauptdurchströmungsrichtung aufweist.

8. Verfahren nach einem der Ansprüche 1 bis 7, wobei auf den Support-Monolith ein Washcoat aus, bezogen auf das keramische Material des Supports, dem gleichen oder einem anderen keramischen Material aufgetragen wird

9. Verfahren nach Anspruch 8, wobei die Menge das auf dem Support befindlichen Waschcoats ≤ 20 Gew.-% beträgt, bezogen auf die Gesamtmenge des Supports

10. Verfahren nach einem der Ansprüche 1 bis 9, wobei das Metall des Hydroformylierungskatalysatorsystems Eisen, Iridium, Ruthenium, Cobalt oder Rhodium ist, vorzugsweise Cobalt oder Rhodium.

11. Verfahren nach einem der Ansprüche 1 bis 10, wobei der organische Phosphor-enthaltende Ligand des Hydroformylierungskatalysatorsystems die allgemeine Formel (VI)
R' - A- R"-A- R''' ( VI)
aufweist, wobei R', R" und R''' jeweils organische Reste sind, mit der Maßgabe das R' und R''' nicht identisch sind, und beide A jeweils eine brückende -O-P(-O)₂-Gruppe sind, wobei zwei der drei Sauerstoffatome -O-jeweils an Rest R' und den Rest R''' gebunden sind.

12. Verfahren nach einem der Ansprüche 1 bis 11, wobei der Stabilisator eine organische Aminverbindung ist, die mindestens eine 2,2,6,6-Tetramethylpiperidineinheit nach Formel (I) enthält:

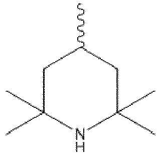

## Claims

1. Process for starting up a hydroformylation reactor containing a heterogenized hydroformylation catalyst system on a support monolith, where the hydroformylation catalyst system comprises a metal of group 8 or 9 of the Periodic Table of the Elements, at least one organic phosphorus-containing ligand, a stabilizer and optionally an ionic liquid, where the process is **characterized in that**
a gaseous feed mixture comprising C2-C8-olefins to be hydroformylated is introduced together with a synthesis gas mixture into the reactor and
the composition of the feed mixture and/or the composition of the synthesis gas mixture is varied at a constant volume flow in a plurality of steps by increasing the proportion of C2-C8-olefins to be hydroformylated in the feed mixture and/or the proportion of synthesis gas in the synthesis gas mixture stepwise, with the proviso that a maximum conversion of the C2-C8-olefins used of from 40 to 90% is not exceeded during the entire startup.

2. Process according to Claim 1, wherein an inert gas is added to the feed mixture and/or the synthesis gas mixture in order to decrease the proportion of C2-C8-olefins and/or to decrease the proportion of synthesis gas and the addition of the inert gas is reduced stepwise correspondingly to the stepwise increase in the proportion of C2-C8-olefins and/or the proportion of synthesis gas.

3. Process according to Claim 1 or 2, wherein inert gas is added to the feed mixture and/or to the synthesis gas mixture in such an amount that the proportion of inert gas in the feed mixture and/or in the synthesis gas mixture is in the range from 70% to 90%.

4. Process according to Claim 3, wherein the inert gas is a noble gas, in particular argon or helium, a C2-C8-alkane or nitrogen.

5. Process according to any of Claims 1 to 4, wherein the support monolith consists of a porous ceramic material selected from the group consisting of a silicate ceramic, an oxidic ceramic, a nitridic ceramic, a carbidic ceramic, a silicidic ceramic and mixtures thereof.

6. Process according to Claim 5, wherein the porous ceramic material of which the support monolith consists is a carbidic ceramic.

7. Process according to Claim 1, wherein the support monolith has one or more channels, preferably through channels, in the main flow direction.

8. Process according to any of Claims 1 to 7, wherein a washcoat of, based on the ceramic material of the support, the same ceramic material or a different ceramic material is applied to the support monolith.

9. Process according to Claim 8, wherein the amount of the washcoat present on the support is ≤ 20% by weight, based on the total amount of the support.

10. Process according to any of Claims 1 to 9, wherein the metal of the hydroformylation catalyst system is iron, iridium, ruthenium, cobalt or rhodium, preferably cobalt or rhodium.

11. Process according to any of Claims 1 to 10, wherein the organic phosphorus-containing ligand of the hydroformylation catalyst system has the general formula (VI)
R'-A-R"-A-R''' (VI),
where R', R" and R''' are each organic radicals, with the proviso that R' and R''' are not identical, and each moiety A is a bridging -O-P(-O)₂- group, where two of the three oxygen atoms -O- are bound in each case to the radical R' and the radical R'''.

12. Process according to any of Claims 1 to 11, wherein the stabilizer is an organic amine compound containing at least one 2,2,6,6-tetramethylpiperidine unit of the formula (I):

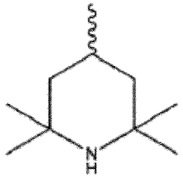

## Revendications

1. Procédé pour la mise en route d'un réacteur d'hydroformylation, qui contient un système de catalyseur d'hydroformylation hétérogénéisé sur un monolithe de support, le système de catalyseur d'hydroformylation comprenant un métal du groupe 8 ou 9 du système périodique des éléments, au moins un ligand organique contenant du phosphore, un stabilisant et éventuellement un liquide ionique, le procédé étant **caractérisé en ce qu'**un mélange d'alimentation gazeux, qui comprend des oléfines en C₂₋₈ devant être hydroformylées, est introduit conjointement avec un mélange de gaz de synthèse dans le réacteur, et la composition du mélange d'alimentation et/ou la composition du mélange de gaz de synthèse, à débit volumétrique constant, sont variées en plusieurs étapes du fait que la proportion d'oléfines en C₂₋₈ devant être hydroformylées dans le mélange d'alimentation et/ou la proportion de gaz de synthèse dans le mélange de gaz de synthèse sont augmentées par étapes,
étant entendu qu'une transformation maximale des oléfines utilisées en C₂₋₈ de 40 à 90 % n'est pas dépassée pendant l'ensemble de la mise en route.

2. Procédé selon la revendication 1, un gaz inerte pour la réduction de la proportion en oléfines en C₂₋₈ et/ou pour la réduction de la proportion en gaz de synthèse étant ajouté au mélange d'alimentation et/ou au mélange de gaz de synthèse et l'ajout du gaz inerte étant réduit par étapes de manière à correspondre à l'augmentation par étapes de la proportion en oléfines en C₂₋₈ et/ou de la proportion en gaz de synthèse.

3. Procédé selon la revendication 1 ou 2, du gaz inerte étant ajouté au mélange d'alimentation et/ou au mélange de gaz de synthèse dans une telle mesure que la proportion du gaz inerte dans le mélange d'alimentation et/ou dans le mélange de gaz de synthèse se situe dans la plage de 70 % à 90 %.

4. Procédé selon la revendication 3, le gaz inerte étant un gaz noble, en particulier l'argon ou l'hélium, un alcane en C₂₋₈ ou l'azote.

5. Procédé selon l'une quelconque des revendications 1 à 4, le monolithe de support étant constitué d'un matériau céramique poreux, qui est choisi dans le groupe constitué par une céramique de silicate, une céramique d'oxyde, une céramique de nitrure, une céramique de carbure, une céramique de siliciure et des mélanges correspondants.

6. Procédé selon la revendication 5, le matériau céramique poreux, qui constitue le monolithe de support, étant une céramique de carbure.

7. Procédé selon la revendication 1, le monolithe de support présentant un ou plusieurs canaux, de préférence continus, dans la direction d'écoulement principal.

8. Procédé selon l'une quelconque des revendications 1 à 7, un revêtement d'imprégnation, composé du même matériau céramique ou d'un matériau céramique différent que le matériau céramique du support, étant déposé sur le monolithe de support.

9. Procédé selon la revendication 8, la quantité du revêtement d'imprégnation se trouvant sur le support étant ≤ 20 % en poids, par rapport à la quantité totale du support.

10. Procédé selon l'une quelconque des revendications 1 à 9, le métal du système de catalyseur d'hydroformylation étant le fer, l'iridium, le ruthénium, le cobalt ou le rhodium, de préférence le cobalt ou le rhodium.

11. Procédé selon l'une quelconque des revendications 1 à 10, le ligand organique contenant du phosphore du système de catalyseur d'hydroformylation présentant la formule générale (VI)
**R'-A-R"-A-R'''** **(VI),**
R', R" et R''' étant à chaque fois des radicaux organiques, étant entendu que R' et R''' ne sont pas identiques, et les deux A étant à chaque fois un groupe pontant -O-P(-O)₂, deux des trois atomes d'oxygène -O-étant à chaque fois liés au radical R' et au radical R'''.

12. Procédé selon l'une quelconque des revendications 1 à 11, le stabilisant étant un composé de type amine organique, qui contient au moins un motif 2,2,6,6-tétraméthylpipéridine selon la formule (I) :
